# EUROPÄISCHE PATENTSCHRIFT

(11) **EP 1 317 959 B1**
(45) Veröffentlichungstag und Bekanntmachung des Hinweises auf die Patenterteilung: **14.11.2007**
(21) Anmeldenummer: 02027073.2
(22) Anmeldetag: 03.12.2002
(51) Int. Cl.: B01J 23/89, B01J 37/03, C07C 29/141, C07C 29/145

(54) **Verfahren zur Herstellung von Ruthenium/Eisen/Kohlenstoffträger-Katalysatoren**
Process for producing carbon-supported ruthenium/iron-catalysts
Procédé de préparation de catalyseurs de ruthénium/fer sur un support de carbone

(30) Priorität: 07.12.2001 DE 10160144; 31.07.2002 DE 10235064
(43) Veröffentlichungstag der Anmeldung: 11.06.2003
(73) Patentinhaber: BASF Aktiengesellschaft, 67056 Ludwigshafen (DE)
(72) Erfinder: Gerlach, Till, Dr., 67071 Ludwigshafen (DE); Göbbel, Hans-Georg, Dr., 67169 Kallstadt (DE); Funke, Frank, Dr., 68161 Mannheim (DE); Ebel, Klaus, Dr., 68623 Lampertheim (DE); Schwab, Ekkehard, Dr., 67434 Neustadt (DE); Unverricht, Signe, Dr., 68169 Mannheim (DE); Körner, Reinhard, Dr., 67227 Frankenthal (DE); Lobree, Lisa, Dr., Philadelphia, PA 19130 (US)

(56) Entgegenhaltungen:
- EP-A- 0 071 787
- EP-A- 0 993 866
- WO-A-99/43430
- WO-A-03/039743
- FR-A- 2 791 672
- US-A- 3 284 517

## Beschreibung

Die vorliegende Erfindung betrifft ein Verfahren zur Herstellung von mit Eisen dotierten Ruthenium/Kohlenstoffträger-Katalysatoren sowie deren Verwendung zur selektiven Flüssigphasenhydrierung von Carbonylverbindungen zu den entsprechenden Alkoholen, insbesondere zur Hydrierung von Citral zu Geraniol bzw. Nerol oder von Citronellal zu Citronellol.

Aus dem Stand der Technik sind verschiedene Verfahren zur Herstellung von Katalysatoren bekannt. Die Verfahren unterscheiden sich im wesentlichen durch die verwendeten Vorstufen der aktiven Komponenten oder durch die Art der Abscheidung der aktiven Komponenten auf dem Kohlenstoff-Träger.

EP 071 787 offenbart Ruthenium/Eisen/Kohle-Hydrierkatalysatoren sowie deren Herstellung und Verwendung zur selektiven Hydrierung ungesättigter Carbonylverbindungen. Die Herstellung des verwendeten Ru/Fe/Kohlenstoff-Katalysators erfolgt durch Tränkung von Aktivkohlepulver mit Rutheniumchlorid-Lösung, Trocknung und anschließendem Vermischen mit Eisenoxid. Reduziert wird der Katalysator mit Wasserstoff bei 500°C.

Die Verwendung von Chloriden bereitet jedoch technische Probleme, da Chlorid stark korrosiv wirkt. So muss beim Tränken und Trocknen der aktiven Komponenten in teueren, korrosionsbeständigen Apparaturen gearbeitet werden. Bei der Reduktion entsteht HCl, die den Reduktionsofen schädigen kann und es kann Chlorid auf dem Katalysator verbleiben, das bei Einsatz des Katalysators in der Hydrierung zu Korrosion im Produktionsreaktor führen kann.

Verwendet man statt der Chloride die Nitratsalze des Rutheniums, so kann dies zu einer Sicherheitsproblematik führen, da Nitrat/Kohle-Gemische explosiv sein können. Ein weiterer Nachteil des beschriebenen Verfahrens ist die sequentielle Dotierung mit Fe₂O₃, die einen zusätzlichen Verfahrensschritt erfordert.

WO 99/43430 bezieht sich auf Katalysatoren für Oxidationsreaktionen in der Flüssigphase, insbesondere in sauren Medien. Die Katalysatoren enthalten einen mit einem Edelmetall beladenen Kohlenstoffträger. Ein Beispiel beschreibt die Katalysatorherstellung durch Zugabe einer gemeinsamen Lösung von Eisen und Platin zu einer Akivkohle-Slurry, Anheben des pH-Werts durch Zugabe von NaOH, Filtrieren, Trocknen und Heizen in einem Wasserstoff/Argon-Strom bei 690 °C.

Aus dem Stand der Technik sind ebenfalls verschiedene Hydrierverfahren für α,β-ungesättigte Carbonylverbindungen bekannt. Mit den beschriebenen Verfahren und den eingesetzten Katalysatoren ist es jedoch schwierig, hohe Selektivitäten der entsprechenden Alkohole zu erhalten. Bei der Hydrierung von Citral können beispielsweise neben der Aldehydgruppe auch die olefinischen Doppelbindungen hydriert werden oder nur die zur Aldehydgruppe konjugierte Doppelbindung, so dass neben den ungesättigten Alkoholen Geraniol bzw. Nerol auch Nebenprodukte wie Citronellol oder Citronellal entstehen können.

In M.M. Paulose et al, J. Oil Technol. Assoc. India 1974, 6, 88-91, wird ein kontinuierliches Festbettverfahren zur selektiven Hydrierung von Citral beschrieben. Als Katalysatoren wurden Kupfer/Chrom/Cadmium-Katalysatoren verwendet. Um ausreichende Citral-Umsätze zu erzielen, muss bei hohen Temperaturen von 170 bis 250°C gearbeitet werden. Es wird eine maximale Geraniol/Nerol-Ausbeute von 66,4 % bei 225 bis 235°C und annähernd Vollumsatz von Citral erzielt. Die Citronellol-Ausbeute lag bei 14,8 %. Auch bei niedrigeren Umsätzen von ca. 44 % lag die Ausbeute des unerwünschten Citronellols noch bei 7,6 %, die Citronellol-Selektivität somit bei ca. 17 %. Die von Paulose et al. angegebenen Selektivitäten zu Citronellol sind für ein wirtschaftliches Verfahren zur Hydrierung von Citral zu hoch.

Aufgabe der vorliegenden Erfindung war es, ein verbessertes Verfahren zur Herstellung eines Ruthenium/Eisen/Kohlenstoffträger-Katalysators, insbesondere für die Selektivhydrierung von olefinisch ungesättigten Carbonylverbindungen zu den entsprechend ungesättigten Alkoholen, zu entwickeln, ohne die oben beschriebenen Nachteile.

Der Katalysator sollte eine verbesserte Katalysatoraktivität und Langzeitstabilität aufweisen sowie auch insbesondere bei der Hydrierung von Citral zu Geraniol/Nerol zu hohen Citralumsätzen und gleichzeitig niedrigen Citronellol-Selektivitäten führen.

Bei der Herstellung des Katalysators sollte der Einsatz korrosiver Einsatzstoffe wie Chloridsalze bzw. explosiver Zwischenprodukte wie nitratgetränkte Kohlen vermieden werden.

Erfindungsgemäß gelöst wurde die Aufgabe durch ein Verfahren zur Herstellung eines Ruthenium/Kohlenstoffträger-Katalysators, enthaltend neben 0,1 bis 10 Gew.-% Ruthenium auf einem Kohlenstoffträger 0,1 bis 5 Gew.-% Eisen, durch
a) Einbringen des Trägers in Wasser
b) Simultane Zugabe der katalytisch aktiven Komponenten in Form von Lösungen ihrer Metallsalze
c) gleichzeitige Auffällung der katalytisch akiven Komponenten auf den Träger durch Zugabe einer Base
d) Abtrennen des Katalysators von der wässrigen Phase der Trägersuspension
e) Trocknen des Katalysators
f) Reduktion des Katalysators im Wasserstoffstrom bei 400 bis 600°C
g) Ausbau des Katalysators unter schwer-entflammbaren Flüssigkeiten oder passivieren des Katalysators mit Sauerstoff.

Die Schritte (b) und (c) können nach dem erfindungsgemäßen Verfahren sowohl nacheinander als auch simultan durchgeführt werden.

Ein weiterer Gegenstand der Erfindung ist die Verwendung der nach dem erfindungsgemäßen Verfahren hergestellten Ruthenium/Eisen/Kohlenstoffträger-Katalysatoren zur selektiven Flüssigphasenhydrierung von Carbonylverbindungen der allgemeinen Formel I, 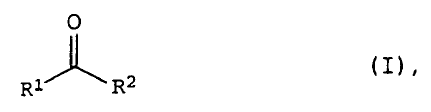 wobei
- R¹, R²: jeweils unabhängig voneinander gleich oder verschieden sein können und Wasserstoff oder einen gesättigten oder einen ein- oder mehrfach ungesättigten geradkettigen oder verzweigten gegebenenfalls substituierten C₁-C₂₀-Alkylrest, einen gegebenenfalls substituierten Arylrest oder eine gegebenenfalls substituierte heterocyclische Gruppe darstellen
zu den entsprechenden Alkoholen der allgemeinen Formel II 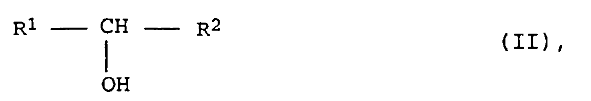
wobei R¹ und R² die oben angegebene Bedeutung haben.

Als Carbonylverbindungen können sowohl gesättigte als auch olefinisch ungesättigte Carbonylverbindungen eingesetzt werden.

Unter einem gesättigten oder ein oder mehrfach ungesättigten geradkettigen oder verzweigten C₁-C₂₀-Alkylrest versteht man, wenn nichts anderes angegeben, einen Methyl-, Ethyl-, Propyl-, Isopropyl-, n-Butyl-, i-Butyl-, t-Butyl, Pentyl-, Hexyl-, Heptenyl-, Octyl-, Nonyl-, Decyl-, 1-Propenyl-, 2-Propenyl-, 2-Methyl-2-propenyl-, 1-Pentenyl-, 1-Methyl-2-Pentenyl-, Isopropenyl-, 1-Butenyl-, Hexenyl-, Heptenyl-, Octenyl-, Nonenyl- oder einen Decenylrest bzw. die den weiter unten aufgeführten eingesetzten Verbindungen entsprechenden Reste.

Unter einem Arylrest versteht man einen Benzyl-, Phenyl- oder Naphthylrest.

Unter einer heterocyclischen Gruppe versteht man beispielsweise einen Pyridin-, Pyrimidin-, Pyridazin-, Pyrazin-, Piperazin-, Imidazol-, Furan-, Oxazol-, Isothiazol-, Isoxazol-, 1,2,3-Triazol- oder 1,2,4-Triazol-, Thiazol-, Thiophen- oder Indolring.

Substituenten können Methyl-, Ethyl-, Propyl-, i-Propyl-, Butyl-, t-Butyl-, Fluor-, Chlor-, Brom-, Iod-, Nitro- oder Aminoreste bedeuten.

Als gesättigte Carbonylverbindungen werden beispielsweise 3,7-Dimethyloctan-1-al und seine Isomeren, Tetrahydrogeranylaceton, Hexahydrofarnesylaceton, 6-Methylheptanon oder Isovaleraldehyd eingesetzt.

Als olefinisch ungesättigte Carbonylverbindungen können beispielsweise Citronellal, H-Geranylaceton, H-Nerolidol, Methylvinylketon, Mesityloxid, Pseudoionen, Dihydrofarnesylaceton, Lysmeral, Methylhexenon, insbesondere bevorzugt Citronellal oder aber α,β-ungesättigte Carbonylverbindungen, beispielsweise Acrolein, Methacrolein, Crotonaldehyd, Prenal, Farnesal oder Citral, insbesonders bevorzugt Citral eingesetzt werden.

Unter den unter Schritt (g) des erfindungsgemäßen Verfahrens genannten schwer-entflammbaren Flüssigkeiten versteht man Flüssigkeiten mit einem Flammpunkt größer als 80°C, bevorzugt größer 100°C, beispielsweise Wasser, Geraniol, Pentandiol, Ethylenglykol oder Nerol bzw. Gemische davon, insbesonders bevorzugt Geraniol oder Nerol bzw. Mischungen davon.

Überraschenderweise führt die simultane Auffällung der Metallsalze der aktiven Komponenten Ruthenium und Eisen zu einer verbesserten Katalysatoraktivität, -selektivität und -standzeit. Durch die Ausfällung der Metalle in Form ihrer Hydroxide werden die im Stand der Technik aufgeführten Probleme der Korrosion bzw. der Explosionsgefahr vermieden.

Als Metallsalze der aktiven Komponenten Ruthenium und Eisen können die Chloride, Nitrate, Nitrosylnitrate, Acetate, Oxide, Hydroxide, Acetylacetonate, bevorzugt die Chloride und Nitrate, eingesetzt werden.

Mit dem erfindungsgemäßen Verfahren kann der Katalysator sowohl als Festbett- als auch als Suspensionskatalysator hergestellt werden.

Unter den Kohlenstoffträger-Materialien versteht man beispielsweise Graphite, Ruße oder Aktivkohle, vorzugsweise jedoch Aktivkohle z.B. NORIT SX Plus^{®}. Je nachdem, ob der Katalysator als Suspensions- oder Festbettkatalysator hergestellt werden soll, wird das Kohlenstoffträgermaterial in pulvriger Form oder in Form von Strängen, Kugeln, Splitt etc. eingesetzt. Der Kohlenstoffträger kann vor seiner Dotierung vorbehandelt werden, etwa durch Oxidation mit Salpetersäure, Sauerstoff, Wasserstoffperoxid, Salzsäure, etc.

Die Herstellung des erfindungsgemäßen Katalysators wird im einzelnen wie folgt durchgeführt:

Zur Herstellung eines Suspensionskatalysators wird der Kohlenstoffträger in Wasser suspendiert (Schritt (a)) und die so hergestellte Trägersuspension wird entweder ohne weitere Vorbehandlung, d.h. ohne die Einstellung eines bestimmten pH-Wertes, oder durch Einstellen eines pH-Wertes kleiner als 6 mit einer Säure, beispielsweise HNO₃, oder größer als 8 mit einer Base, beispielsweise NaOH, für das weitere Verfahren verwendet.

In Schritt (b) erfolgt die simultane Zugabe der aktiven Komponenten Ruthenium und Eisen in Form von Lösungen ihrer Metallsalze. Die Zugabe erfolgt bevorzugt bei erhöhter Temperatur der Suspension, insbesonders bevorzugt bei einer Temperatur zwischen 50 und 95°C, insbesonders bevorzugt bei einer Temperatur von 70 bis 90°C. Anschließend wird zur Ausfällung der katalytisch aktiven Komponenten auf den Träger eine Base, beispielsweise Na₂CO₃, NaHCO₃, (NH₄)₂CO₃, NH₃, Harnstoff, NaOH, KOH oder LiOH, bevorzugt NaOH, langsam hinzugefügt und der pH-Wert zwischen 6 und 14, bevorzugt auf 8 bis 12, besonders bevorzugt auf 9, angehoben ((c)). Die Zugabe der Base erfolgt bevorzugt bei erhöhter Temperatur, insbesonders bevorzugt bei einer Temperatur zwischen 50 und 95°C, bevorzugt bei einer Temperatur von 70 bis 90°C. Die Zugabe der Base kann auch gleichzeitig mit der Zugabe der Metallsalzlösung erfolgen, etwa um den pH-Wert der Suspension konstant zu halten, bevorzugt auf einen pH-Wert von 8 bis 14, besonders bevorzugt von 9. Da nach der Fällung Ruthenium und Eisen im wesentlichen als Hydroxide vorliegen, werden Chlorid- bzw. Nitratanionen bei der an die Fällung angeschlossene Waschung und Abtrennung des Katalysators von der wässrigen Phase ((d)) auf einen unproblematisch niedrigen Gehalt ausgewaschen.

Der Filterkuchen wird anschließend unter Vakuum oder Inertgas getrocknet (e) und dann wird der Katalysator in einem Wasserstoffstrom, eventuell verdünnt mit einem Inertgas wie Stickstoff, zwischen 400 und 600°C reduziert (f). Zum Abschluss wird der Katalysator dann nach Abkühlung auf Temperaturen unterhalb von 40°C z.B. unter Wasser oder einer schwer-entflammbaren Flüssigkeit ausgebaut oder durch Überströmen eines verdünnten Sauerstoffstroms (etwa 1 % Sauerstoff in einem Inertgas wie Stickstoff) passiviert (g).

Die Herstellung der Festbettkatalysatoren erfolgt analog dem für den Suspensionskatalysator beschriebenen Verfahren, wobei in Schritt (a) anstelle des pulverförmigen Trägermaterials Stränge, Kugeln, Splitt etc. eingesetzt werde. Die charakteristischen Längen dieser Formkörper (Durchmesser, Länge etc.) liegen in der Regel oberhalb von 1 mm. Bei der Dispergierung der Stränge in Wasser ist darauf zu achten, dass deren mechanische Beanspruchung möglichst gering gehalten wird, um den Abrieb zu minimieren. Vorteilhafterweise werden die Stränge vor Einsatz in der Katalysatorsynthese mit Wasser gewaschen, um schwach anhaftende feinteilige Kohlepartikel zu entfernen.

Die erfindungsgemäßen Katalysatoren enthalten im allgemeinen 0,1 bis 10 Gew.-% Ruthenium auf einem Kohlenstoffträger, vorzugsweise auf Aktivkohle.

Die BET-Oberfläche der Katalysatoren beträgt, entsprechend der zur Herstellung verwendeten Kohlenstoffträger, etwa 100 bis 1500, vorzugsweise etwa 800 bis 1200 m²/g. Die Teilchengröße der Ruthenium-Kristallite liegt im Wesentlichen unterhalb von 10 nm und entspricht damit den aus der Literatur für Ruthenium/Kohlenstoff-Katalysatoren bekannten Werten.

Die Angaben über die im Katalysator enthaltenen Gew.-% an Ruthenium und Eisen beziehen sich in der vorliegenden Anmeldung immer auf die Trockenmasse des Katalysators.

Besondere Bedeutung hat der erfindungsgemäß hergestellte Katalysator für die selektive Hydrierung von Carbonylverbindungen, bevorzugt für die selektive Hydrierung von ungesättigten Carbonylverbindungen, insbesonders bevorzugt für die Hydrierung von Citral zu Geraniol bzw. Nerol oder von Citronellal zu Citronellol.

Der erfindungsgemäß hergestellte Katalysator hydriert mit überraschend hoher Selektivität die Aldehyd-Gruppe der Carbonylverbindung.

Das Hydrierverfahren kann sowohl kontinuierlich als auch diskontinuierlich in Suspension oder im Festbett durchgeführt werden. Besonders vorteilhaft ist die kontinuierliche Fahrweise.

Für die Suspensions- oder Festbettvariante bieten sich übliche Reaktorkonzepte an, wie sie z.B. in Ullmann's Encyclopedia of Industrial Chemistry, Sixth Edition, 2000 Electronic Release, beschrieben sind.

Das kontinuierliche bzw. diskontinuierliche Suspensionsverfahren kann beispielsweise wie in EP 947 493 bzw. US 5,939,589 beschrieben durchgeführt werden. Der Katalysator wird sowohl in der diskontinuierlichen als auch in der kontinuierlichen Suspensionsfahrweise in feinverteilter Form eingesetzt, wobei die Teilchengröße kleiner 1 mm, bevorzugt zwischen 1 und 100 µm, ist.

Für die Festbettvariante wird der Katalysator in für Festbettkatalysatoren üblichen Formen beispielsweise in Strang-, Splittform, Tabletten- oder Kugelform eingesetzt. Typische Strangdurchmesser liegen zwischen 1 und 5 mm, die Stranglängen liegen bei 1 bis 20 mm. Der Reaktor kann in Riesel- oder Sumpffahrweise betrieben werden.

Die Reaktion wird sowohl bei der Suspensionsfahrweise als auch bei der Festbettvariante drucklos oder unter einem Druck von 1 bis 200 bar, bevorzugt 10 bis 100 bar, insbesonders bevorzugt bei 20 bis 50 bar, durchgeführt. Die Temperaturen liegen zwischen 25 und 200°C, bevorzugt zwischen 60 und 100°C. Die Reaktion kann sowohl mit als auch ohne Lösungsmittel durchgeführt werden. Als Lösungsmittel können niedere Alkohole wie Methanol, Ethanol oder Isopropylalkohol eingesetzt werden. Weiterhin kann bei Bedarf eine organische Base wie Trimethylamin zugesetzt werden.

Die Hydrierung der Carbonylverbindung an den erfindungsgemäß hergestellten Katalysatoren wird vorzugsweise in Gegenwart eines tertiären Amins durchgeführt.

Prinzipiell sind jegliche tertiäre Amine geeignet, so dass es auf deren chemische Natur, sofern sie aufgrund funktioneller Gruppen nicht anderweitig mit den Reaktionspartnern reagieren können, nicht ankommt.

Als Amine kommen beispielsweise die in EP 071 787 genannten in Frage.

Die Menge der Amine beträgt vorzugsweise 1 bis 5 Gew.-% der Menge der eingesetzten Carbonylverbindung.

Die nachfolgenden Beispiele sollen die Erfindung näher erläutern, ohne sie jedoch darauf zu beschränken.

### Herstellung von Suspensionskatalysatoren

### Beispiel 1

A) 100 g Aktivkohle wurden mit 500 ml konz. HNO₃ versetzt und im 1-L-Kolben bei 80°C 6 h lang gerührt. Nach Abkühlung wurde filtriert und der Filterkuchen mit 10 l dest. Wasser gewaschen.
   Die feuchte Kohle wurde wieder in den Rührkessel gegeben, mit 2,5 l Wasser suspendiert und unter Rückflusskühlung auf 80°C erhitzt. Dann wurde innerhalb von 120 min unter Rühren eine Lösung aus 13,11 g Rutheniumchlorid und 5,15 g Eisenchlorid in 375 ml Wasser zugetropft. Der pH-Wert der Suspension betrug nach Zugabe der Metallsalzlösung 1,4. Durch Zugabe von 1-M-Natronlauge wurde dann der pH-Wert durch langsames Zutropfen auf 9 angehoben; hierzu wurden etwa 400 ml NaOH verbraucht. Anschließend wurde 1 h nachgerührt und dann abgekühlt. Der Katalysator wurde auf eine Glasfilternutsche überführt, mit insgesamt 40 l Wasser gewaschen und im Vakuum-trockenschrank für 6 h bei 80°C getrocknet. Das getrocknete Pulver wurde dann in einem Drehkugelofen in einem Strom aus 70 % H₂ und 30 % N₂ bei 500°C für 3 h reduziert. Nach Ende der Reduktion wurde unter Stickstoff abgekühlt und mit einer Gasmischung aus 1 % Sauerstoff in Stickstoff passiviert. Der fertige Katalysator hatte einen Chloridgehalt von unter 0,05 Gew.-%. Weiterhin werden folgende Gehalte (Gew.-%) bestimmt: Na: 2,8, Ru: 5,2, Fe: 1,1.
B) Es wurde gearbeitet wie in A beschrieben, jedoch wurde anstelle von Ruthenium- und Eisenchlorid Rutheniumnitrosylnitrat und Eisen-(III)-Nitrat verwendet. Der fertige Katalysator hatte einen Ruthenium-Gehalt von 5,1 Gew.-%, einen Eisen-Gehalt von 1,1 Gew.-%, einen Nitrat-Gehalt < 0,01 Gew.-% und einen Na-Gehalt von 2,1 Gew.-%.
C) Es wurde gearbeitet wie in A beschrieben, jedoch wurden geringere Ruthenium- und Eisengehalte auf die Aktivkohle aufgebracht. Der fertige Katalysator hatte einen Ruthenium-Gehalt von 2,8 Gew.-%, einen Eisen-Gehalt von 0,54 Gew.-%, einen Chlorid-Gehalt von 0,02 Gew.-% und einen Na-Gehalt von 3,8 Gew.-%.
D) 110 g der Aktivkohle Norit SX Plus^{®} wurden ohne weitere Vorbehandlung in einen Rührkolben mit 2 l Wasser gegeben, suspendiert und unter Rückfluss auf 80°C erhitzt. Dann wurde der pH-Wert durch Zugabe von wässriger NaOH (1 mol/l) auf 9 angehoben. Innerhalb einer Stunde wurde dann bei 80°C 300 ml einer Lösung von Ruthenium-Nitrosylnitrat und Eisennitrat (Konzentration entsprechend 5,85 g Ru und 1,17 g Fe) zugetropft, gleichzeitig wurde durch simultane Zugabe von wässriger NaOH der pH-Wert auf ca. 9 gehalten. Es wurde eine Stunde bei 80°C nachgerührt und dann abgekühlt. Die kalte Suspension wurde filtriert und mit 40 l Wasser gewaschen, danach im Vakuum-Trockenschrank 16 h bei 80°C getrocknet und wie unter A beschrieben reduziert und passiviert. Der Katalysator hatte einen Ru-Gehalt von 5,0 Gew.-%, einen Fe-Gehalt von 1,0 Gew.-% und einen Na-Gehalt von 0,036 Gew.-%.
E) Vergleichsbeispiel: Es wurde ein Katalysator nach der in EP 0071787 angegebenen Vorschrift durch Tränkung der Aktivkohle mit Ru-Chloridlösung, Trocknung, Vermischung mit Eisenrot, Reduktion bei 500°C und Ausbau unter Wasser hergestellt.

### Testung der unter A bis D hergestellten Katalysatoren

### a) Kontinuierliche Suspensionshydrierung

Die kontinuierliche Suspensionshydrierung wurde in einem gepackten Blasensäulenreaktor mit Produktrückführung und Wasserstoffkreisgas (entsprechend EP 947 493) durchgeführt.

Es wurde eine Flüssigkeitsmischung aus 70 Vol.-% Citral (E/Z ≈ 1), 27 Vol.-% Methanol und 3 Vol.-% Trimethylamin hydriert. Flüssigkeit und Gas wurden über eine gepackte Blasensäule (Packungsvolumen: 143 ml, Packungsdurchmesser: 27 mm) mit einer Durchflussgeschwindigkeit von jeweils ca. 120 l/h gefahren. Der Citral-Umsatz konnte durch Variation der Parameter Druck, Temperatur oder Edukt-Zuflussrate eingestellt werden. Der Druck wurde zwischen 20 und 40 bar und die Temperatur zwischen 80 und 100°C variiert.

### b) Diskontinuierliche Hydrierung

Die diskontinuierliche Hydrierung wurde in einem Autoklaven der Fa. Medimex durchgeführt. Das flüssige Edukt (250 ml einer Mischung aus 70 Vol.-% Citral (E/Z = 1), 27 Vol.-% Methanol und 3 Vol.-% Trimethylamin) und der Katalysator (2,5 g) wurden vor Versuchsbeginn in den Autoklaven überführt. Anschließend wurde bei Normaldruck mit Stickstoff gespült. Nach dem vollständigen Entfernen von Luft aus dem Reaktionsraum wurde die Stickstoffzufuhr beendet, der Autoklav entspannt und auf Wasserstoff umgestellt. Dann wurde Wasserstoff in den Autoklaven geleitet und der gewünschte Reaktionsdruck eingestellt. Unterdessen wurde auf die erforderliche Reaktionstemperatur geregelt. Nach Erreichen der Temperatur und des Drucks wurde der Rührer eingeschaltet und damit die Reaktion gestartet. Während der Reaktion konnten über ein Steigrohr Proben aus dem Reaktionsraum genommen werden. Es wurde bei einer Temperatur von 100°C und einem Wasserstoffdruck von 50 bar hydriert.

### Ergebnis

Die in Tabelle 1 dargestellten Ergebnisse zeigen, dass die erfindungsgemäßen Katalysatoren A bis D wesentlich aktiver und selektiver sind als der Vergleichskontakt. Insbesondere bei hohen Umsätzen wird deutlich mehr Geraniol/Nerol und deutlich weniger Citronellol gebildet. Gegenüber dem Vergleichskatalysator muss, um hohe Umsätze zu erreichen, weniger Trockenmasse an Katalysator eingesetzt werden (Beispiel A und B) bzw. es kann eine geringere Beladung mit Edelmetall eingestellt werden (Beispiel C). Außerdem desaktivieren die erfindungsgemäßen Katalysatoren langsamer als der Vergleichskontakt.

In Tabelle 2 ist der Aktivitätsvergleich des erfindungsgemäßen Katalysators A mit dem Vergleichskatalysator aus einem diskontinuierlichen Hydrierversuch gezeigt. Nach 50 min erreicht der Katalysator A bereits annähernd Vollumsatz, während der erfindungsgemäße Kontakt lediglich 50 % des Citrals umgesetzt hat.
F) Ein entsprechend Beispiel 1A) hergestellter Katalysator wurde zur kontinuierlichen Suspensionshydrierung von Citronellal (3,7-Dimethylokt-7-en-1-al) zu Citronellol (3,7-Dimethylokt-7-en-1-ol) eingesetzt. Hierbei ist die Bildung des vollhydrierten Produktes 3,7-Dimethyloktan-1-ol so weit wie möglich zu vermeiden. Als Edukt wurde eine Mischung aus 70 % Citronellal, 27 % Methanol und 3 % Trimethylamin eingesetzt. Bei einer Reaktionstemperatur von 80°C, einem Druck von 20 bar, einer Eduktzufuhr von 2,2 ml/min, einer Katalysatormenge von 40 g (trocken) wurde nach 142 h ein Citronellal-Umsatz von 94 %, eine Citronellol-Selektivität von 97,3 % und eine Selektivität zu 3,7-Dimethyloktan-1-ol von nur 1,4 % erhalten. Der Katalysator zeichnete sich durch eine hohe Aktivität und Langzeitstabilität und eine hohe Selektivität zu Citronellol auch bei hohen Citronellal-Umsätzen aus.

**Tabelle 1**

| Katalysator | Trockenmasse | Zeit | Temperatur | Druck | Edukt-Zufuhr | Umsatz | Selektivität | | |
|---|---|---|---|---|---|---|---|---|---|
| | g | h | °C | bar | ml/min | % | Geraniol/Nerol / % | Citronellol / % | Rückstand / % |
| A | 40 | 150 | 80 | 20 | 2,2 | 92,4 | 96,8 | 1,2 | 2,0 |
| | | 225 | 80 | 40 | 2,2 | 96,1 | 97,6 | 1,6 | 0,7 |
| | | 280 | 90 | 40 | 2,2 | 98,6 | 94,6 | 4,3 | 1,1 |
| | | 400 | 80 | 20 | 0,7 | 98,6 | 95,2 | 3,0 | 1,8 |
| | | 440 | 80 | 20 | 2,2 | 89,8 | 98,1 | 1,3 | 0,6 |
| B | 40 | 170 | 80 | 20 | 2,2 | 91,1 | 97,2 | 1,7 | 1,1 |
| | | 240 | 80 | 40 | 2,2 | 95,8 | 96,5 | 2,1 | 1,4 |
| | | 310 | 90 | 40 | 2,2 | 96,6 | 96,0 | 2,3 | 1,7 |
| | | 350 | 80 | 20 | 2,2 | 88,8 | 97,0 | 1,7 | 1,3 |
| C | 40 | 260 | 80 | 20 | 2,2 | 81,1 | 97,5 | 1,2 | 1,3 |
| | | 330 | 90 | 40 | 2,2 | 92,4 | 96,7 | 1,3 | 2,0 |
| | | 430 | 100 | 40 | 2,2 | 95,5 | 95,6 | 3,0 | 1,4 |
| D | 40 | 167 | 80 | 20 | 2,2 | 90,9 | 96,1 | 1,4 | 2,5 |
| | | 210 | 80 | 40 | 2,2 | 95,7 | 95,7 | 2,5 | 1,8 |
| E | 40 | 140 | 80 | 20 | 1,5 | 84,4 | 94,6 | 2,8 | 2,6 |
| | | 170 | 80 | 40 | 1,5 | 93,2 | 94,1 | 4,3 | 1,6 |
| | | 815 | 80 | 40 | 2,3 | 75,5 | 96,3 | 1,8 | 1,9 |

**Tabelle 2**

| Zeit / min | Umsatz / % | |
|---|---|---|
| | Vergleichskatalysator E | Katalysator A |
| 0 | 0 | 0 |
| 30 | 50 | 98 |
| 60 | 76 | 100 |
| 90 | 90 | 100 |
| 120 | 97 | 100 |
| 150 | 100 | 100 |
| 180 | 100 | 100 |

### Herstellung von Festbettkatalysatoren

### Beispiel 2

62 g Aktivkohlestränge (Supersorbon SX 30 der Fa. Lurgi, Durchmesser 3 mm, Oberfläche ca. 1000 m² g⁻¹) wurden mit 400 ml VE-Wasser in einem Rührkessel vorgelegt und unter leichtem Rühren und Rückflusskühlung auf 80°C erhitzt. Eine Lösung aus 8,13 g Rutheniumchlorid und 3,19 g Eisenchlorid wurde bei 80°C innerhalb von 60 min zugetropft. Dann wurde durch Zugabe von 1 M Natronlauge der pH-Wert auf 9 angehoben und eine Stunde nachgerührt. Der Katalysator wurde auf eine Glasfilternutsche überführt, mit 10 l VE-Wasser gewaschen und anschließend im Vakuum-Trockenschrank 6 Stunden bei 80°C getrocknet. Dann wurde in einem Reduktionsofen für 3 Stunden bei 500°C in einem Gasgemisch aus Wasserstoff und Stickstoff (50/4) reduziert, auf Raumtemperatur abgekühlt und mit einem Gasgemisch aus 1 % Sauerstoff in Stickstoff passiviert.

### Testung der Katalysatoren

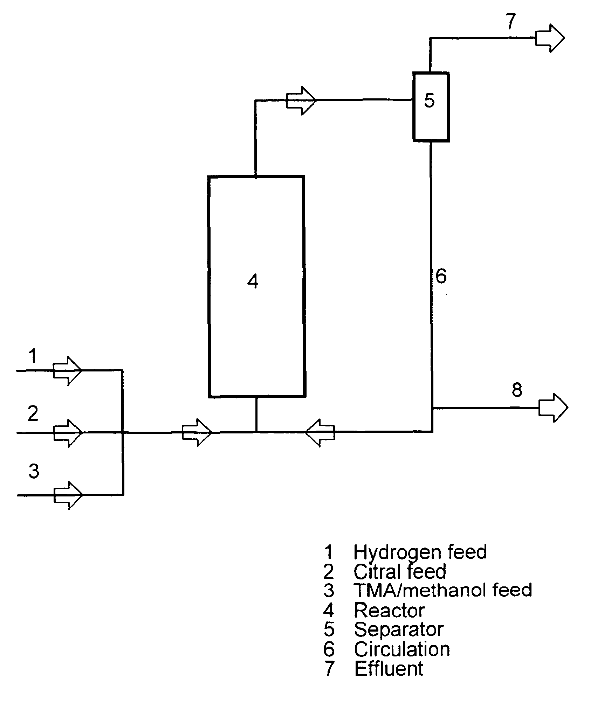

### Siehe dazu auch Figur 1

| | | |
|---|---|---|
| Versuchsparameter: | Druck | 40 bar |
| | Temperatur | 50 bis 70°C |
| | | |
| Reaktordaten: | Länge: | 470 mm |
| | Durchmesser: | 20 mm |
| | Volumen: | 150 ml |
| | Kat.-Volumen | 100 ml |

### Beschreibung

Citral und eine Methanol-Trimethylamin-Lösung werden separat durch Kolbenpumpen mit Waagensteuerung zugepumpt. Das Verhältnis Citral/Methanol/Trimethylamin beträgt etwa 70/27/3. Der Wasserstoff-Zulauf wird über einen Massendurchflussregler dosiert. Der Reaktor wird in Sumpffahrweise bei 50 bis 70°C betrieben. Vom Abscheider-Sumpf werden 80 % des Reaktor-Austrags mittels Kolbenpumpe rückgeführt und durch einen Schwebekörperdurchflussmesser kontrolliert. Der übrige Produkt-Anteil wird über ein Druckhalteventil ausgetragen.

### Allgemeine Durchführung

Der Katalysator wird in den Reaktor eingebaut und reduziert. Dabei wird der Kontakt drucklos mit 13 Nl/h Wasserstoff auf 120°C geheizt. Nach 1 Stunde wird abgekühlt. Anschließend werden Citral und Trimethylamin/Methanol-Gemisch getrennt dem Reaktor zugeführt. Die Parameter sind den einzelnen Versuchen zu entnehmen. Die Analytik erfolgt mit einer 30 m DBWAX 0,32 mm 0,25 µm Säule (80°C - 3°C/min - 230°C - 20 min).

### Beispiel 3

Festbettkatalysator aus Beispiel 2 mit Rückführung: 100 ml (41,1 g) Katalysator wurden in den Reaktor eingebaut und nach der allgemeinen Vorschrift aktiviert. Bei einem Wasserstoffdruck von 40 bar (24 Nl/h) wurden die Zuläufe wie folgt eingestellt: Citral (2, 60 g/h, Reinheit 98 %), Methanol + 10 % TMA (3, 25,5 g/h), Rückstrom (6, 240 g/h). Bei einer Reaktortemperatur von 75°C wurde bei einem Umsatz von 95,61 % (Laufzeit: 713 h) eine Selektivität zu Nerol/Geraniol von 95,22 % erreicht (Verhältnis Ger/Ner: 1,20). Nebenprodukte: Selektivität Citronellol: 1,80 %, Selektivität Nerol-Isomere: 1,70 %.

## Patentansprüche

1. Verfahren zur Herstellung eines Ruthenium/Eisen/Kohlenstoffträger-Katalysators, enthaltend neben 0,1 bis 10 Gew.-% Ruthenium auf einem Kohlenstoffträger 0,1 bis 5 Gew.-% Eisen, durch
a) Einbringen eines Trägers in Wasser
b) Simultane Zugabe der katalytisch aktiven Komponenten Ruthenium und Eisen in Form von Lösungen ihrer Metallsalze
c) gleichzeitige Auffällung der katalytisch aktiven Komponenten auf den Träger durch Zugabe einer Base
d) Abtrennen des Katalysators von der wässrigen Phase der Trägersuspension
e) Trocknen des Katalysators
f) Reduktion des Katalysators im Wasserstoffstrom bei 400 bis 600°C
g) Ausbau des Katalysators unter schwer-entflammbaren Flüssigkeiten oder passivieren des Katalysators mit einem verdünnten Sauerstoffstrom

2. Verfahren gemäß Anspruch 1, **dadurch gekennzeichnet, dass** der hergestellte Katalysator ein Suspensionskatalysator ist.

3. Verfahren gemäß Anspruch 1, **dadurch gekennzeichnet, dass** der hergestellte Katalysator ein Festbettkatalysator ist.

4. Verfahren gemäß einem der Ansprüche 1 bis 3, **dadurch gekennzeichnet, dass** die Schritte (b) und (c) bei einer Temperatur von 50 bis 95°C durchgeführt werden.

5. Verfahren gemäß einem der Ansprüche 1 bis 4, **dadurch gekennzeichnet, dass** die Schritte (b) und (c) sowohl gleichzeitig als auch nacheinander erfolgen können.

6. Verfahren gemäß einem der Ansprüche 1 bis 5, **dadurch gekennzeichnet, dass** die katalytisch aktiven Komponenten in Form ihrer Chloride, Nitrate, Nitrosylnitrate, Acetate, Oxide, Hydroxide oder Acetylacetonate eingesetzt werden.

7. Verfahren gemäß einem der Ansprüche 1 bis 6, **dadurch gekennzeichnet, dass** der Kohlenstoffträger durch Oxidation mit HNO₃, Sauerstoff, Wasserstoffperoxid oder Salzsäure vorbehandelt werden kann.

8. Verfahren gemäß einem der Ansprüche 1 bis 7, **dadurch gekennzeichnet, dass** zum Ausfällen der katalytisch aktiven Komponenten auf den Träger als Base Na₂CO₃, NaHCO₃, (NH₄)₂CO₃, NH₃, Harnstoff, NaOH, KOH oder LiOH verwendet wird.

9. Verfahren gemäß einem der Ansprüche 1 bis 8, **dadurch gekennzeichnet, dass** zum Ausfällen der katalytisch aktiven Komponenten NaOH verwendet wird.

10. Verwendung des nach einem Verfahren gemäß einem der Ansprüche 1 bis 9 hergestellten Katalysators zur selektiven Flüssigphasenhydrierung von Carbonylverbindungen der allgemeinen Formel I 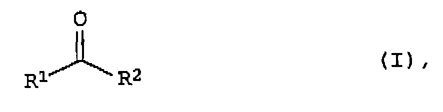 wobei
R¹, R² jeweils unabhängig voneinander gleich oder verschieden sein können und Wasserstoff oder einen gesättigten oder einen ein- oder mehrfach ungesättigten geradkettigen oder verzweigten gegebenenfalls aubstituierten C₁-C₂₀-Alkylrest, einen gegebenenfalls substituierten Arylrest oder eine gegebenenfalls substituierte heterocyclische Gruppe darstellen,
zu den entsprechenden Alkoholen der allgemeinen Formel II 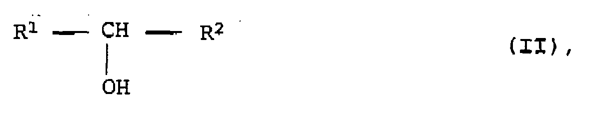
wobei R¹ und R² die oben angegebene Bedeutung haben.

11. Verwendung gemäß Anspruch 10, **dadurch gekennzeichnet, dass** die Carbonylverbindung eine α,β-ungesättigte Carbonylverbindung ist.

12. Verwendung gemäß einem der Ansprüche 10 oder 11, **dadurch gekennzeichnet, dass** die Carbonylverbindung Citral ist.

13. Verwendung gemäß einem der Ansprüche 10 bis 12, **dadurch gekennzeichnet, dass** die Carbonylverbindung Citronellal ist.

## Claims

1. A process for preparing a ruthenium/iron catalyst supported on carbon, comprising besides 0.1 to 10% by weight of ruthenium on a carbon support 0.1 to 5% by weight of iron, by:
a) suspending a support in water,
b) simultaneously adding the catalytically active components ruthenium and iron in the form of solutions of their metal salts,
c) simultaneously precipitating the catalytically active components onto the support by addition of a base,
d) separating the catalyst from the aqueous phase of the support suspension,
e) drying the catalyst,
f) reducing the catalyst in a hydrogen stream at from 400 to 600°C, and
g) conditioning the catalyst under low flammability liquids or passivating the catalyst with a dilute oxygen stream.

2. The process according to claim 1, wherein the catalyst prepared is a suspension catalyst.

3. The process according to claim 1, wherein the catalyst prepared is a fixed bed catalyst.

4. The process according to any of claims 1 to 3, wherein steps (b) and (c) are carried out at a temperature in the range from 50 to 95°C.

5. The process according to any of claims 1 to 4, wherein steps (b) and (c) may be either simultaneous or else in succession.

6. The process according to any of claims 1 to 5, wherein the catalytically active components are used in the form of their chlorides, nitrates, nitrosyl nitrates, acetates, oxides, hydroxides or acetylacetonates.

7. The process according to any of claims 1 to 6, wherein the carbon support can be pretreated by oxidation with HNO₃, oxygen, hydrogen peroxide or hydrochloric acid.

8. The process as claimed any of claims 1 to 7, wherein the base used to precipitate the catalytically active components on the support is Na₂CO₃, NaHCO₃, (NH₄)₂CO₃, NH₃, urea, NaOH, KOH or LiOH.

9. The process according to any of claims 1 to 8, wherein NaOH is used to precipitate the catalytically active components.

10. The use of a catalyst prepared by a process according to any of claims 1 to 9 for the selective liquid phase hydrogenation of carbonyl compounds of the general formula I 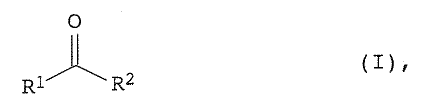 where
R¹ and R² are identical or different and are each independently hydrogen or a saturated or mono- or polyunsaturated straight chain or branched substituted or unsubstituted C₁-C₂₀-alkyl radical, an unsubstituted or substituted aryl radical or an unsubstituted or substituted heterocyclic group,
to give the corresponding unsaturated alcohols of the general formula II 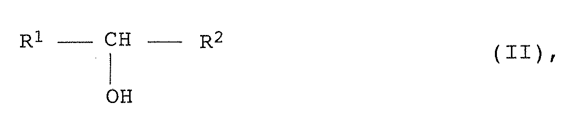 where R¹ and R² are each as defined above.

11. The use according to claim 10, wherein the carbonyl compound is an α,β-unsaturated carbonyl compound.

12. The use according to claim 10 or 11, wherein the carbonyl compound is citral.

13. The use according to any of claims 10 to 12, wherein the carbonyl compound is citronellal.

## Revendications

1. Procédé de préparation d'un catalyseur de ruthénium/fer sur un support de carbone, contenant, outre de 0,1 à 10% en poids de ruthénium sur un support de carbone, de 0,1 à 5% en poids de fer, par
a) introduction d'un support dans de l'eau,
b) addition simultanée des composants catalytiquement actifs de ruthénium et de fer sous forme de solutions de leurs sels métalliques,
c) précipitation simultanée des composants catalytiquement actifs sur le support par addition d'une base,
d) séparation du catalyseur et de la phase aqueuse de la suspension de support,
e) séchage du catalyseur,
f) réduction du catalyseur dans un courant d'hydrogène à une température de 400 à 600°C,
g) conditionnement du catalyseur dans des liquides peu inflammables ou passivation du catalyseur avec un courant d'oxygène dilué.

2. Procédé suivant la revendication 1, **caractérisé en ce que** le catalyseur préparé est un catalyseur en suspension.

3. Procédé suivant la revendication 1, **caractérisé en ce que** le catalyseur préparé est un catalyseur à lit fixe.

4. Procédé suivant l'une quelconque des revendications 1 à 3, **caractérisé en ce que** les étapes (b) et (c) sont entreprises à une température de 50 à 95°C.

5. Procédé suivant l'une quelconque des revendications 1 à 4, **caractérisé en ce que** les étapes (b) et (c) peuvent avoir lieu tant en même temps que successivement.

6. Procédé suivant l'une quelconque des revendications 1 à 5, **caractérisé en ce que** les composants catalytiquement actifs sont mis en oeuvre sous la forme de leurs chlorures, nitrates, nitrosylnitrates, acétates, oxydes, hydroxydes ou acétylacétonates.

7. Procédé suivant l'une quelconque des revendications 1 à 6, **caractérisé en ce que** le support de carbone est prétraité par oxydation par du HNO₃, de l'oxygène, du peroxyde d'hydrogène ou de l'acide chlorhydrique.

8. Procédé suivant l'une quelconque des revendications 1 à 7, **caractérisé en ce que**, pour la précipitation des composants catalytiquement actifs sur le support, on utilise en tant que base du Na₂CO₃, du NaHCO₃, du (NH₄)₂CO₃, du NH₃, de l'urée, du NaOH, du KOH ou du LiOH.

9. Procédé suivant l'une quelconque des revendications 1 à 8, **caractérisé en ce que**, pour la précipitation des composants catalytiquement actifs, on utilise du NaOH.

10. Utilisation du catalyseur préparé par un procédé suivant l'une quelconque des revendications 1 à 9 pour l'hydrogénation sélective en phase liquide de composés carbonyle de formule générale I 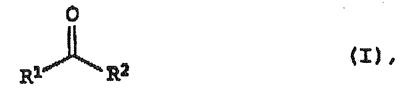 dans laquelle
R¹, R² peuvent être à chaque fois identiques ou différents et représenter indépendamment l'un de l'autre de l'hydrogène ou un radical alkyle en C₁ à C₂₀ saturé ou une ou plusieurs fois insaturés, à chaîne linéaire ou ramifiée et éventuellement substitués, un radical aryle éventuellement substitué ou un groupe hétérocyclique éventuellement substitué,
pour obtenir les alcools correspondants de formule générale II 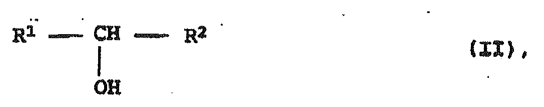
dans laquelle R¹ et R² ont les significations indiquées plus haut.

11. Utilisation suivant la revendication 10, **caractérisée en ce que** le composé carbonyle est un composé carbonyle α,β-insaturé.

12. Utilisation suivant l'une quelconque des revendications 10 ou 11, **caractérisée en ce que** le composé carbonyle est du citral.

13. Utilisation suivant l'une quelconque des revendications 10 à 12, **caractérisée en ce que** le composé carbonyle est du citronellal.
